# EUROPEAN PATENT APPLICATION

(11) **EP 2 623 607 A1**
(43) Date of publication of application: **07.08.2013**
(21) Application number: 12153565.2
(22) Date of filing: 02.02.2012
(51) Int. Cl.: C12P 7/10, C12P 7/26, C12P 7/40, C12M 1/00

(54) **Method and system for production of a target chemical from a cellulosic material**

(71) Applicant: Sekab E-Technology AB, 891 26 Örnsköldsvik (SE)
(72) Inventor: Wingren, Anders, 891 34 ÖRNSKÖLDSVIK (SE)
(74) Representative: Wirén, Anders

(57) **Abstract**

The present invention relates to a method of production of a target chemical from a cellulosic material, comprising the steps of:
a) pretreating the cellulosic material under overpressure;
b) releasing pressure to obtain pretreated cellulosic material and flash steam;
c) hydrolyzing and fermenting the pretreated cellulosic material to obtain a liquid containing the target chemical; and
d) distilling the liquid from step c) to obtain a first target chemical stream and a first stillage stream,
wherein at least part of the flash steam from step b) is supplied as direct steam in the distillation of step d).

## Description

### Technical field

The present invention relates to an improved method and a system for production of a target chemical from a cellulosic material. In particular it relates to an energy efficient method for production of a target chemical from a cellulosic material.

### Background

Biorefineries producing green products from renewable resources offer an alternative to oil refineries based on dwindling supplies of petroleum and permit a move towards improved energy security. Lignocellulosic materials from forestry and agriculture are attractive as feedstock, since they are abundant, relatively inexpensive, and are considered to be non food crops.. Lignocellulose consists mainly of lignin and two classes of polysaccharides, cellulose and hemicellulose. The polysaccharides can be hydrolyzed to sugars and be converted to various fermentation products, through well established fermentation techniques. The fermentation products can be purified, concentrated and/or isolated through e.g. distilling processes well known in the art. However, due to the chemical and structural properties of the lignocellulose, polysaccharides are trapped inside the crosslinking structure of the biomass. Thus, pretreatment of the biomass have been described as an important process in order to remove and modify the surrounding matrix of lignin and hemicellulose, prior to hydrolysis of the polysaccharides into their component sugars (Zheng et al. Int J Agric & Biol. Eng, 2009). There are a number of pre-treatment methods known in the art. One such pre-treatment method is steam explosion which has been described as one of the most cost-effective pre-treatment methods (Zheng et al. Int J Agric & Biol. Eng, 2009).

### Summary

The process of production of target chemicals, such as e.g. bioalcohols, from lignocellulosic biomass can involve several different steps, such as a pretreatment step, a hydrolysis step, a fermentation step and a distillation step. In the pretreatment step mainly the hemicellulose is degraded and the lignocellulosic biomass is converted into a form for which enzymatic cellulose hydrolysis is more effective. The sugars generated during the hydrolysis steps can be fermented into a mash comprising the target chemical. The target chemical can be isolated from the mash and purified through one or more distillation steps. The first distillation column where the distillation of the mash is performed is some times referred to as the mash column.

In some pretreatment methods, such as steam explosion, the biomass is treated with steam at high temperature and high pressure. These kinds of pretreatment methods are typically terminated by a rapid release of pressure, whereupon the biomass undergoes an explosive decompression and a flash steam is generated. The present invention is partially based upon the insight that the energy efficiency in the process of production of target chemicals from lignocellulosic biomass can be substantially increased if the energy generated in the flash steam is reused.

The inventors have further realized that it is particularly suitable to reuse the energy in the flash steam by supplying at least a part of the flash steam as direct steam in the distillation step. This can be achieved by introduction of at least a part of the flash steam into the mash column such that energy is transferred to the mash and thereby, less external energy needs to be supplied in the distillation process.

Thus, in a first aspect the invention relates to a method of production of a target chemical from a cellulosic material, comprising the steps of:
a) pretreating the cellulosic material under overpressure;
b) releasing pressure to obtain a pretreated cellulosic material and flash steam;
c) hydrolyzing and fermenting the pretreated cellulosic material to obtain a liquid containing the target chemical; and
d) distilling the liquid from step c) to obtain a first target chemical stream and a first stillage stream,
wherein at least part of the flash steam from step b) is supplied as direct steam in the distillation of step d).

A second aspect of the invention relates to a system for production of a target chemical from a cellulosic material, comprising:
a) a pressure reactor for pretreating the cellulosic material, said pressure reactor comprising an inlet and an outlet;
b) a flashing chamber for recovery of flash steam from the material from the pressure reactor, said flashing chamber comprising an inlet being connected to the outlet of the pressure reactor, a flash steam outlet and a cellulosic material outlet;
c) an arrangement for hydrolysis and fermentation of the material from the flashing chamber to produce a fermentation broth containing the target chemical, said arrangement comprising an inlet connected to the cellulosic material outlet of the flashing chamber and an outlet;
d) a distillation apparatus comprising a distillation column for separating the liquid into a target chemical fraction and a stillage fraction by means of steam heating, said distillation apparatus comprising a fermentation broth inlet connected to the outlet of the arrangement for hydrolysis and fermentation, a flash steam inlet connected to the flash steam outlet of the flashing chamber, a target chemical outlet and a stillage outlet, wherein the distillation apparatus is arranged to route flash steam from the flash steam inlet into the distillation column for direct heating of the distillation.

### Brief description of the figures

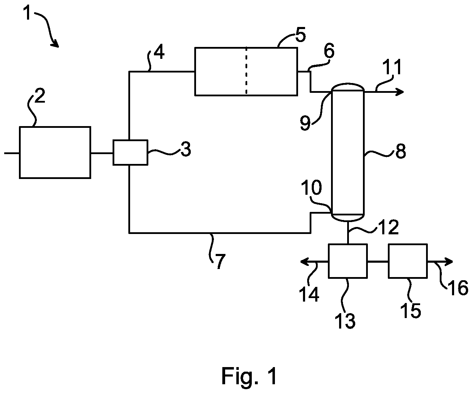

Figure 1 shows a system for production of a target chemical from a cellulosic material, comprising: a pressure reactor for pretreating the cellulosic material, a flashing chamber for recovery of flash steam from the material from the pressure reactor, an arrangement for hydrolysis and fermentation of the material from the flashing chamber to produce a fermentation broth and a distillation apparatus comprising a distillation column for separating the liquid into a target chemical fraction and a stillage fraction. The system is arranged to route flash steam from the flashing chamber into the distillation column for direct heating of the distillation.
Figure 2 shows a series of additional units which are arranged in between a cellulosic material outlet of the flashing chamber and an inlet of the arrangement for hydrolysis and fermentation.
Figure 3 shows the distilling apparatus connected to a second distilling apparatus in which the target chemical fraction can be further purified.

### Detailed description

Some pretreatment methods, involves treatment of the biomass with steam at high temperature and high pressure followed by a rapid release of pressure, whereupon the biomass undergoes an explosive decompression and a flash steam is generated. As mentioned above, the present invention is partially based upon the insight that the energy efficiency in a process of production of a target chemical from lignocellulosic biomass can be increased if the energy in the flash steam is reused.

The inventors have further realized that it is particularly suitable to reuse the energy in the flash steam by supplying at least a part of the flash steam as direct steam in the distillation step. This can be achieved by introduction of at least a part of the flash steam into the distilling column where it transfers its energy to the mash present in the distilling column, and thereby less external energy needs to be supplied in the distillation process. By supplying the flash steam as direct steam the invention circumvents the need of a heat exchanger, such as a reboiler, in order to transfer the energy to the mash. This feature therefore solves several different problems compared to if the flash steam would have been introduced into a heat exchanger. Most importantly, lignin and other organic molecules present in the mash would clog and foul the heat exchanger which would stall the production process. To be able to run such a process continuously more than one heat exchanger connected to the distillation column would probably be needed so that at least one heat exchanger can be used while another is cleaned. Therefore, using the energy in the flash steam indirectly in a heat exchanger would probably be a much more expensive solution than the method according to the present invention where the flash steam is supplied as direct steam.

Therefore, a first aspect of the invention relates to a method of production of a target chemical from a cellulosic material, comprising the steps of:
a) pretreating the cellulosic material under overpressure;
b) releasing pressure to obtain pretreated cellulosic material and flash steam;
c) hydrolyzing and fermenting the pretreated cellulosic material to obtain a liquid containing the target chemical; and
d) distilling the liquid from step c) to obtain a first target chemical stream and a first stillage stream,
wherein at least part of the flash steam from step b) is supplied as direct steam in the distillation of step d).

Another advantage of using the flash steam as direct steam is that the mash (i.e. the liquid containing the target chemical obtained in step c) does not need to be filtered prior to distillation. If the energy instead would have been transferred to the mash via a heat exchanger, e.g. a reboiler, solid material comprising lignin present in the mash would clog the heat exchanger, provided that said solid material has not been removed prior to the distillation step. Therefore, in the method according to the present invention, the dry matter content of the mash can be higher compared to methods utilizing heat exchangers for transfer of the energy from the flash steam to the distillation process. Thus, in one embodiment of the invention the dry matter content of the liquid containing the target chemical obtained in c) and being subjected to distillation in step d) is above 5 % (w/v) such as above 10 % (w/v).In a preferred embodiment the dry matter content of the liquid containing the target chemical obtained in c) and being subjected to distillation in step d) is 5 %-25 % (w/v), such as 5%-20% w/v). Since solids does not have to be removed from the liquid containing the target chemical obtained in step c) prior to distillation, the process according to the present invention can be simpler and cheaper compared to methods where the energy in the flash steam is supplied indirectly via a heat exchanger. Thus in one embodiment of the method solids is not removed from the liquid containing the target chemical obtained in c) prior to the distilling of said liquid in d).

Optionally, the first target chemical stream obtained in step d) can be subjected to a second distillation process to further purify and increase the concentration of the target chemical. Thus, in one embodiment the method further comprises a step d2) of subjecting the first target chemical stream obtained in step d) to a second distillation to obtain a second target chemical stream and a second stillage stream.

The flash steam comprises several different organic molecules and therefore, it is less suitable to introduce flash steam as direct steam in the second distillation column. If flash steam is introduced as direct steam in the second distillation column it can contaminate the first target chemical stream obtained in step d) which might affect the purity of the second target chemical stream obtained in step d2). Accordingly, in one embodiment flash steam from step b) is not supplied as direct steam in the distillation of step d2).

Introduction of flash steam as direct steam in the distillation of step d) will cause a dilution of the liquid containing the target chemical obtained in c). The grade of dilution is dependent on the amount of flash steam supplied in the distillation of step d) in relation to the volume of the liquid containing the target chemical obtained in c). In one embodiment the liquid containing the target chemical obtained in c) is diluted 2-20 %, such as 5-15%, by the supplement of flash steam in the distillation of step d).

In the distilling process a certain amount of energy (e.g. x mega joules) is needed to be introduced in order to isolate a certain volume of ethanol (e.g. y liters) from the mash. Introduction of flash steam as direct steam in the distillation of step d) will reduce the demand for supply of external energy to the distillation process. For example if x mega joules is needed in the distilling process to isolate y liters of ethanol and 10 % of the energy used in the distilling step is derived from the flash steam, only 90 % of the external energy has to be supplied compared to if no flash steam would have been introduced. How much the supply of additional external energy can be reduced depends on the amount of flash steam being supplied as well as on the heat energy present in the flash steam. In one embodiment at least 5 % of the energy used for distilling in step d) is derived from the flash steam supplied as direct steam in the distillation of step d). In one embodiment at least 10 % of the energy used for distilling in step d) is derived from the flash steam supplied as direct steam in the distillation of step d). In a preferred embodiment 10-50 % of the energy used for distilling in step d) is derived from the flash steam supplied as direct steam in the distillation of step d). In one embodiment the flash steam obtained in step b) has a pressure in a range between 1 and 20 bar, preferably in a range between 1 and 10 bar. In another embodiment the flash steam obtained in step b) has a temperature in a range between 100°C and 180 °C.

The flash steam contains volatile organic matter derived from the cellulosic material and the present inventors have realized that by introducing the flash steam as direct steam in the distillation, the volatile organic matter mixes with the stillage stream in step d) such that at least part of the organic matter from the flash steam become part of the stillage stream. The inventors have realized that the organic molecules present in the flash steam can be converted to a valuable product by using the stillage stream as a substrate in a biological treatment process.

Many of the organic compounds, such as e.g. acetic acid, HMF, furfural, levulinic acid and formic acid in the flash steam are toxic to fermentation organisms used for converting the sugars to the target chemical. Such compounds may also be toxic to the enzymes if enzymatic hydrolysis is used. Another advantage of the method is thus that the toxic compounds are moved past the steps of hydrolysis and fermentation and mixed with the mash in the distillation. Consequently, the toxic compounds of the flash steam may not inhibit the hydrolysis or fermentation, but may still be treated together with other residual organic matter after the distillation without any extra separation steps. This is particularly beneficial when the hydrolysis is enzymatic as in such case, none of the typical organic inhibitors are produced in the hydrolysis step and thus a substantial proportions of all the inhibitors generated in the method may be passed past the fermentation step. Thus, the first aspect may, at least in some of its embodiments, be regarded as a detoxification method.

To facilitate the biological treatment process, solids can be removed prior to the biological treatment. Accordingly, in one embodiment the method according to the present invention further comprises a step e) of subjecting the stillage stream to biological treatment to lower its content of organic matter. Optionally solids are separated from the stillage stream prior to the biological treatment. Said biological treatment can be selected from an aerobic and/or an anaerobic treatment. In one embodiment the aerobic treatment comprises the cultivation of a microorganism capable of producing hydrolytic enzymes. This is a particularly suitable application since production of hydrolytic enzymes on site has the potential to reduce the cost of hydrolytic enzymes used in the process. The filamentous fungi Hypocrea jecorina is particularly suitable for production of hydrolytic enzymes. Therefore, in a preferred embodiment the microorganism is a filamentous fungi, such as Hypocrea jecorina. Another suitable biological treatment of the stillage involves production of biogas. Hence, in one embodiment the anaerobic treatment comprises biogas production. In combination with the biological treatment, or as an alternative to the biological treatment, other suitable waste water treatments can also be used.

The method according to the present inventions is suitable for production of a host of different target chemicals which can be derived from cellulosic material. Preferably the target chemical should be able to be produced through a fermentation process and to be purified or isolated through a distilling process. In one embodiment the target chemical is selected from alcohols, acids, alkanes, alkenes, aromatics, aldehydes and ketones. In one embodiment the target chemical is selected from ethanol, butanol and succinic acid. In a preferred embodiment the target chemical is ethanol.

In one embodiment the hydrolyzing in step c) is acid hydrolysis and in another embodiment the hydrolyzing in step c) is enzymatic hydrolysis. In one embodiment, the hydrolyzing and fermentation in step c) is a simultaneous saccharification and fermentation (SSF) of the pretreated lignocellulosic material. A SSF process refers to a process in which enzymatic hydrolysis and fermentation is performed simultaneously in a fermentor. Thus, in a SSF process, fermentable saccharides are prepared directly in a fermentor by enzymatic hydrolysis of the pretreated lignocellulosic material, and the resulting saccharides are converted into a fermentation product. Further, the fermentation may be a consolidated bioprocess (CBP), in which the biocatalyst that converts the monosaccharides also produces the enzymes that hydrolyze the pretreated lignocellulosic material. In another embodiment, the hydrolysate that is subjected to fermentation is obtained from a separate, preceding step of enzymatic hydrolysis. Consequently, the enzymatic hydrolysis and the fermentation may be performed as two separate process steps (separate hydrolysis and fermentation, SHF). This may e.g. be advantageous if the fermentation reaction and the enzymatic reaction have different optimal temperatures. As an example, the temperature during enzymatic hydrolysis may be kept higher than the temperature during fermentation, thus facilitating the use of thermophilic enzymes.

In one embodiment fermentation is performed by a fermenting organism and the fermenting organism can for example be bacteria and/or yeast. In one embodiment the fermenting agent is bacteria from the genus Zymomonas or Escherichia. In another embodiment the fermenting organism is yeasts from the genus Saccharomyces, Pichia or Candida. Preferably, the fermenting organism may be wild type, mutant or recombinant Saccharomyces cerevisiae. Using S. cerevisiae for producing a fermentation product is advantageous since S. cerevisiae is well established with regard to industrial fermentation and provides for a high product yield.

A suitable pretreatment method according to the present invention is "steam explosion". Steam explosion pretreatment methods are divided in "uncatalyzed steam explosion" and "catalyzed steam explosion". In uncatalyzed steam explosion no catalyst is used. The biomass is rapidly heated by high-pressure steam (without any catalyst) for a period of time to promote hemicellulose hydrolysis. The process is terminated by swift release of pressure, which renders the biomass to undergo an explosive decompression (Zheng et al. Int J Agric & Biol. Eng, 2009). During this pretreatment, the hemicellulose is hydrolyzed by acids released from the biomass. Catalyzed steam explosion is similar to uncatalyzed steam explosion except that the biomass is impregnated with some acidic or alkaline liquids or gases prior to steam explosion. Both uncatalyzed steam explosion and catalyzed steam explosion are suitable for the method according to the present invention. Accordingly, in one embodiment the cellulosic material is treated with a catalyst and steam prior to or during step a). In one embodiment the catalyst is an acidic chemical such as an acidic liquid or an acidic gas. In one embodiment the acidic chemical is selected from sulfur dioxide, sulfuric acid, sulfurous acid ,carbon dioxide, oxalic acid, acetic acid, and phosphoric acid. Sulfur dioxide is a particularly suitable catalyst since it has been described to require milder and much less expensive reactor material, generates less gypsum, yields more xylose, and produces more digestible substrate with high fermentability compared to sulfuric acid. In one embodiment the catalyst is an alkaline chemical such as an alkaline gas or an alkaline liquid. In one embodiment the alkaline chemical is ammonia. In a different embodiment the cellulosic material is treated with steam during step a) and no catalyst is added in or prior to step a).

Depending on the pretreatment method and the suitable conditions for fermentation and/or hydrolysis, the pH of the cellulosic material in step b) might have to be adjusted prior to the hydrolyzing and fermenting in step c). For example, the biomass slurry from the pretreatment typically has a pH of below 3 if an acidic catalyst is used, which is too low for most enzymatic hydrolyses and fermentations. Therefore, in one embodiment the pH of the pretreated cellulosic material in step b) is adjusted prior to the hydrolyzing and fermenting in step c) to a pH suitable for hydrolysis and/or fermentation. In one embodiment the pH suitable for hydrolysis and/or fermentation is in a range between 4.0 and 7.0.

The release of pressure and obtaining of flash steam in step b) can be performed in several steps such that different fractions of the obtained flash steam are generated. The characteristic of the fractions may vary in terms of amount of heat energy and amount of organic substances and impurities. Depending on the characteristic of the desired target chemical, the mash and other conditions, such as other applications of the recovered steam, different fractions of the flash steam might be more or less suitable for being supplied as direct steam in the distillation step. For example if the target chemical have a boiling point which is similar to the boiling point of impurities being present in some of the fractions it might be suitable to use a fraction which lacks or only comprises low amounts of said impurities. Thus, in one embodiment the releasing of pressure and obtaining of flash steam in step b) is performed in at least two steps, such as in at least three steps or in at least four steps.

Lignocellulosic materials from forestry and agriculture are attractive as feedstock for production of bioalcohols and other green chemicals, since they are abundant, relatively inexpensive, and are not used for food. Furthermore, steam explosion has been described as one of the most cost-effective pre-treatment methods for pre-treatment of lignocellulosic biomass (Zheng et al. Int J Agric & Biol. Eng, 2009). Accordingly, the method according to the present invention is particularly suitable for lignocellulosic biomass. Thus in one embodiment of the invention the cellulosic material is lignocellulosic biomass. Examples of suitable lignocellulosic biomass include wood materials, such as wood chips, or agricultural residues, such as corn cobs, corn stover, oat hulls, sugar cane bagass and straw, (e.g. straw from barley, wheat, oat or rye). The wood material can for example be spruce, pine, birch, oak or eucalyptus. Switch grass, salix and banagrass are examples of other suitable lignocellulosic biomass.

A second aspect of the invention relates to a system for production of a target chemical from a cellulosic material, comprising:
a) a pressure reactor for pretreating the cellulosic material, said pressure reactor comprising an inlet and an outlet;
b) a flashing chamber for recovery of flash steam from the material from the pressure reactor, said flashing chamber comprising an inlet being connected to the outlet of the pressure reactor, a flash steam outlet and a cellulosic material outlet;
c) an arrangement for hydrolysis and fermentation of the material from the flashing chamber to produce a fermentation broth containing the target chemical, said arrangement comprising an inlet connected to the cellulosic material outlet of the flashing chamber and an outlet;
d) a distillation apparatus comprising a distillation column for separating the liquid into a target chemical fraction and a stillage fraction by means of steam heating, said distillation apparatus comprising a fermentation broth inlet connected to the outlet of the arrangement for hydrolysis and fermentation, a flash steam inlet connected to the flash steam outlet of the flashing chamber, a target chemical outlet and a stillage outlet, wherein the distillation apparatus is arranged to route flash steam from the flash steam inlet into the distillation column for direct heating of the distillation.

The various embodiments of the first aspect, as well as their benefits, apply to the second aspect *mutatis mutandis.* However, certain embodiments of the second aspect are anyway discussed below in some detail.

In one embodiment the system further comprises a unit for biological treatment of the stillage to reduce its organic matter content, said unit being connected to the stillage outlet of the distillation apparatus. In one embodiment the unit is adapted to anaerobic treatment and comprises a biogas outlet and a purified stillage outlet.

In the connection between the stillage outlet of the distillation apparatus and the unit for biological treatment, a device for separation of solids from the stillage may be arranged. Such a device may for example be a centrifuge or a decanter. It may also be a filtering device comprising one or more filter(s) or screen(s).

After the pretreatment and prior to hydrolysis and fermentation, it can be beneficial to adjust the conditions of the pretreated cellulosic material such that the conditions are more suitable for hydrolysis and/or fermentation. Normally the temperature in the pretreatment is much higher than the optimal temperature for the hydrolysis and/or fermentation, and therefore a heat exchanger can be arranged in between the cellulosic material outlet of the flashing chamber and the inlet of the arrangement for hydrolysis and fermentation. Other parameters that might be adjusted prior to hydrolysis/fermentation include pH and conductivity. Accordingly, in one embodiment at least one additional unit is arranged in between the cellulosic material outlet of the flashing chamber and the inlet of the arrangement for hydrolysis and fermentation such that the cellulosic material leaving the flashing chamber passes through the at least one additional unit before it reaches the arrangement for hydrolysis and fermentation. Preferably the at least one additional unit is/are one or more units selected from a buffer vessel, a unit for pH adjustment and a heat exchanger. In some cases it can also be preferable to separate a part of the liquid fraction from the pretreated material. For example, the hydrolysis might be more effective if hemisugars and inhibitors present in the liquid fraction is removed prior to the hydrolysis/fermentation step. In some cases, sugar might be needed for cultivation of yeast or other microorganisms and thus it might be preferred to isolate a part of the sugars prior to fermentation. Therefore in one embodiment the at least one additional unit is a unit for separating a part of the liquid fraction. In one embodiment the unit for separating a part of the liquid fraction comprises at least one filter, centrifuge or decanter.

In one embodiment at least one additional unit is arranged in between the flash steam outlet of the flashing chamber and the flash steam inlet of the distillation apparatus such that the flash steam leaving the flashing chamber passes through the at least one additional unit before it reaches the distillation apparatus. For example, if sulfur dioxide or sulfuric acid is used as a catalyst, the flash steam can comprise sulfur compounds. For both economical and environmental reasons, it can be beneficial to collect and preferably reuse the sulfur compounds. For example the sulfur compounds can be reused as catalysts in the pretreatment. Sulfur compounds can for example be collected by passing the flash steam leaving the flashing chamber through a sulfur recovering unit. Sometimes it can also bee beneficial to remove liquid droplets from the flash steam. This can bee achieved by passing the flash steam, leaving the flashing chamber, through a demister. Therefore, in one embodiment the at least one additional unit comprises a demister and/or a sulfur recovering unit. Sometimes it can be desirable to increase the purity of the flash steam prior to introduction of it into the mash column, e.g. to prevent contamination of the target chemical. The flash steam can for example be condensed in a steam reformer and be used to generate clean steam in the steam reformer. Accordingly, in an alternative configuration the at least one additional unit comprises a steam reformer. However, such a configuration is less preferred since an extra heat exchanging step adds extra costs for equipment and implies heat losses. Further, in such a configuration the organic matter of the flash steam will not automatically mix with the stillage. Another drawback may be that the organic matter of the flash steam fouls the steam reformer.

Preferably, the flash steam mixes directly with the mash present in the distilling apparatus. Therefore the flash steam inlet of the distillation apparatus is preferably arranged at the bottom of the distillation apparatus. For the same reason it is beneficial if the contact surface between the mash present in the distilling apparatus and the flash steam entering the distilling apparatus is large. For example the diameter of the flash steam inlet can be at least 10 cm, such as at least 20 cm. Preferably the diameter is about 50 cm such as in a range between 30 and 70 cm. In one embodiment the flash steam outlet of the flashing chamber is arranged on the top of said flashing chamber.

Optionally, the first target chemical stream obtained in step d) can be subjected to a second distillation process to further purify and increase the concentration of the target chemical. Thus, in one embodiment the target chemical outlet of the distilling apparatus is connected to a second distillation apparatus via a distillate inlet on the second distilling apparatus. Optionally a heat exchanger such as a reboiler is connected in between the target chemical outlet of the first distilling apparatus and the distillate inlet on the second distilling apparatus. Optionally, the target chemical can be further purified and concentrated by additional distillation processes.

### Detailed description of exemplary embodiments

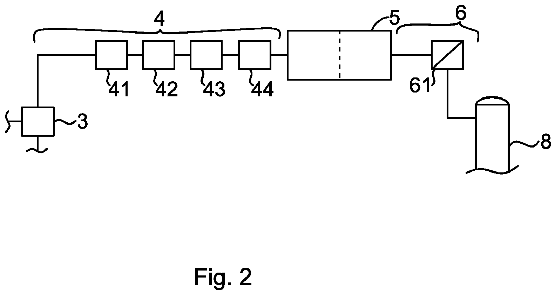

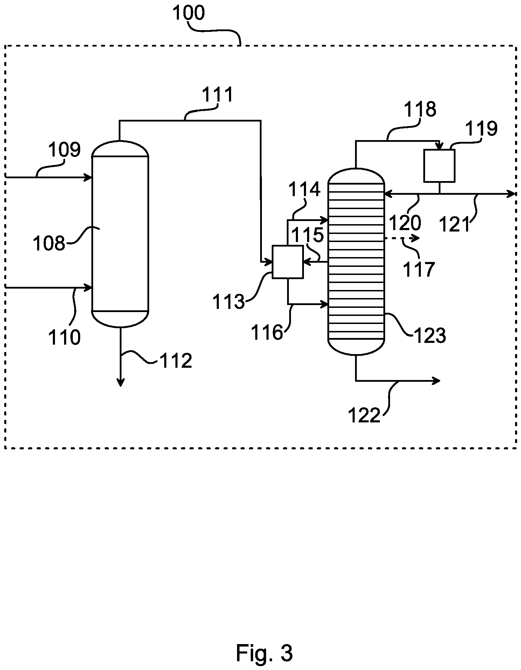

Figure 1 shows a system for production of a target chemical (e.g. ethanol) from a cellulosic material (1). Said system comprises a pressure reactor for pretreating the cellulosic material (2), a flashing chamber (3) for recovery of flash steam, an arrangement for hydrolysis and fermentation (5), a distilling apparatus (8), a device (13) for separation of solids from the stillage and a unit for biological treatment (15). In the pressure reactor (2) lignocellulosic biomass impregnated with sulfur dioxide is initially rapidly heated by high-pressure saturated steam. Thereafter the pressure is swiftly released when the contents of the pressure reactor is emptied into a flashing chamber (3), which renders the biomass to undergo an explosive decompression and a flash steam to be generated. The pretreated biomass formed is transferred to the arrangement for hydrolysis and fermentation (5) via a slurry transfer arrangement (4). In the arrangement for hydrolysis and fermentation (5) the polysaccharides present in the pretreated biomass is hydrolyzed into their component sugars which in turn are converted into ethanol in a SHF or SSF process. The ethanol containing mash produced in the arrangement for hydrolysis and fermentation (5) is transferred to the distilling apparatus (8) via a mash transfer arrangement (6) and enters the distilling apparatus via a mash inlet (9). The distilling apparatus (8) is typically a mash column, which is further described with reference to figure 3 below, The flash steam released from the flashing chamber (3) is transferred to the bottom of the distilling apparatus (8) via a flash steam transfer arrangement (7). The flash steam enters the distilling apparatus (8) via a direct steam inlet (10) and mixes with the mash present in the distilling apparatus (8). The flash steam is thus used for direct heating of the distillation. During the distilling process a stillage stream and distillate stream is formed wherein said distillate stream comprises about 35-40% ethanol. The distillate stream exits the distilling apparatus via distillate outlet (11). The stillage stream exits the distilling apparatus via a stillage outlet (12). The stillage is transferred to the device (13) for separation of solids. The solids leave the device (13) through a solids outlet (14) while the liquid is transferred to the unit for biological treatment (15) and is used for biogas production. The biogas is recovered (not shown) and used, e.g. for electricity production. The liquid purified by the biological treatment exits the unit (15) through a liquid outlet (16) and may, optionally after further purification, be recycled (not shown), e.g. to a step of impregnating the lignocellulosic biomass preceding the steam explosion, to a step of producing steam to be added in the pressure reactor (2) or as process liquid added in connection with the hydrolysis or fermentation in the arrangement (5). Before the cellulosic material enters the arrangement for hydrolysis and fermentation (5), the material may pass a number of additional arrangements to adjust the properties of the pretreated cellulosic material such that it is more suitable for hydrolysis and fermentation. As shown in figure 2, the pretreated biomass is transferred to a buffer vessel (41), a unit for pH adjustment (42) a heat exchanger (43) and a unit for separating a part of the liquid fraction (44).

Since the flash steam is introduced directly into the distilling apparatus, no heat exchanger is needed to heat the mash with the flash steam. If a heat exchanger would have been used for heating the mash in the distilling apparatus (8), solids present in the mash produced in the arrangement for hydrolysis and fermentation (5) would have had to be removed in a separation device (61) in order to prevent clogging and fouling of the heat exchanger. When the flash steam is used for direct heating of the mash, the device for separating solids from the mash (61) can be omitted, which reduces investment and production costs.

Figure 3 shows a system for isolation of ethanol (100). Mash comprising 2-8 % ethanol is introduced into a mash column (108) via a mash inlet (109). Flash steam is introduced into the bottom of the mash column (108) through a direct steam inlet (110). The flash steam mixes with the mash and the energy present in the flash steam is used to distill the mash to form a distillate comprising about 35-40% ethanol, and a stillage stream. To improve the mixing of the flash steam with the mash, the direct steam inlet (110) is arranged at the bottom of the mash column (108). For the same reason the contact surface between the mash present in the mash column (108) and the flash steam entering the mash column is large. This is achieved by a large diameter of the direct steam inlet (110). For example, the diameter of the inlet (110) may be 50 cm. The stillage stream exits the mash column (108) through a stillage outlet (112). The distillate exits the mash column (108) via a distillate outlet (111) and is transferred to a rectifier (117) for further purification and concentration of the ethanol stream. The distillate is first routed to a heat exchanger/reboiler (113), in which energy of the distillate is used to heat a liquid stream from the rectifier (117) routed to the heat exchanger via a connection (115). Steam formed from the liquid stream in the reboiler (113) is returned to the rectifier (117) via a steam inlet (116). After passing the reboiler (113), the distillate is supplied to the rectifier (117) via a distillate inlet (114). Fusel oil, i.e. alcohols having more the two carbon atoms, is diverted from the process via a fusel oil outlet (123) of the rectifier (117). A second stream/water leaves the rectifier (117) via a rectifier bottom outlet (122). The water may, optionally after purification, be recycled (not shown), e.g. to a step of impregnating the lignocellulosic biomass preceding the steam explosion, to a step of producing steam to be added in the pressure reactor (2) or as process liquid added in connection with the hydrolysis or fermentation in the arrangement (5). The distillate leaves the rectifier (117) through a distillate outlet (118) and is transferred to a condenser (119) where the distillate is condensed in to liquid ethanol. A part of the condensed ethanol fraction is returned to the rectifier (117) via an ethanol recycling connection (120). By the distilling process in the rectifier (117) an ethanol stream comprising 92-94% ethanol is obtained (121).

## Claims

1. Method of production of a target chemical from a cellulosic material, comprising the steps of:
a) pretreating the cellulosic material under overpressure;
b) releasing pressure to obtain pretreated cellulosic material and flash steam;
c) hydrolyzing and fermenting the pretreated cellulosic material to obtain a liquid containing the target chemical; and
d) distilling the liquid from step c) to obtain a first target chemical stream and a first stillage stream,
wherein at least part of the flash steam from step b) is supplied as direct steam in the distillation of step d).

2. Method according to claim 1 wherein the dry matter content of the liquid containing the target chemical obtained in c) and being subjected to distillation in step d) is 5-25 %(w/v).

3. Method according to any one of the preceding claims, further comprising the step:
e) subjecting the first stillage stream to biological treatment to lower its content of organic matter, wherein optionally solids are separated from the stillage stream prior to the biological treatment.

4. Method according to any of the preceding claims, wherein the flash steam from step b) contains volatile organic matter derived from the cellulosic material.

5. Method according to claim 4, wherein the flash steam condenses and mixes with the stillage stream in step d) such that part of the organic matter from the flash steam become part of the stillage stream subjected to biological treatment.

6. Method according to any one of the preceding claims, wherein the flash steam obtained in step b) has a pressure in a range between 1 and 20 bar, preferably in a range between 1 and 10 bar.

7. Method according to any one of the preceding claims, wherein the flash steam obtained in step b) has a temperature in a range between 100 °C and 180°C.

8. Method according to any one of the preceding claims wherein the target chemical is selected from alcohols, acids, alkanes, alkenes, aromatics, aldehydes and ketones.

9. Method according to any one of the preceding claims, wherein the cellulosic material is lignocellulosic biomass.

10. Method according to any of the preceding claims wherein 10-50 % of the energy used for distilling in step d) is derived from the flash steam supplied as direct steam in the distillation of step d).

11. System for production of a target chemical from a cellulosic material, comprising:
a) a pressure reactor for pretreating the cellulosic material, said pressure reactor comprising an inlet and an outlet;
b) a flashing chamber for recovery of flash steam from the material from the pressure reactor, said flashing chamber comprising an inlet being connected to the outlet of the pressure reactor, a flash steam outlet and a cellulosic material outlet;
c) an arrangement for hydrolysis and fermentation of the material from the flashing chamber to produce a fermentation broth containing the target chemical, said arrangement comprising an inlet connected to the cellulosic material outlet of the flashing chamber and an outlet;
d) a distillation apparatus comprising a distillation column for separating the liquid into a target chemical fraction and a stillage fraction by means of steam heating, said distillation apparatus comprising a fermentation broth inlet connected to the outlet of the arrangement for hydrolysis and fermentation , a flash steam inlet connected to the flash steam outlet of the flashing chamber, a target chemical outlet and a stillage outlet, wherein the distillation apparatus is arranged to route flash steam from the flash steam inlet into the distillation column for direct heating of the distillation.

12. System according to claim 11 further comprising a unit for biological treatment of the stillage to reduce its organic matter content, said unit being connected to the outlet of the distillation apparatus.

13. System according to claim 12, wherein the unit is adapted to anaerobic treatment and comprises a biogas outlet and a purified stillage outlet.

14. A system according to any of the claims 11-13, wherein at least one additional unit is arranged in between the cellulosic material outlet of the flashing chamber and the inlet of the arrangement for hydrolysis and fermentation such that the cellulosic material leaving the flashing chamber passes through the at least one additional unit before it reaches the arrangement for hydrolysis and fermentation.

15. A system according to claim 14, wherein the at least one additional unit is/are one or more units selected from a buffer vessel, a unit for pH adjustment, a heat exchanger and a unit for separating a part of the liquid fraction.
